# EUROPÄISCHE PATENTSCHRIFT

(11) **EP 2 925 713 B1**
(45) Veröffentlichungstag und Bekanntmachung des Hinweises auf die Patenterteilung: **19.10.2016**
(21) Anmeldenummer: 13792918.8
(22) Anmeldetag: 20.11.2013
(51) Int. Cl.: C07C 29/44, C07C 31/12

(54) **VERFAHREN ZUR HERSTELLUNG VON C4-OXYGENATEN**
METHOD FOR PRODUCING C4 OXYGENATES
PROCÉDÉ DE PRODUCTION D'OXYGÉNATS EN C4

(30) Priorität: 27.11.2012 EP 12194446
(43) Veröffentlichungstag der Anmeldung: 07.10.2015
(73) Patentinhaber: BASF SE, 67056 Ludwigshafen am Rhein (DE)
(72) Erfinder: RÜDENAUER, Stefan, 67549 Worms (DE); EBEL, Klaus, 68542 Heddesheim (DE); PORTA GARCIA, Marta, 68165 Mannheim (DE)
(74) Vertreter: BASF IP Association
(86) Internationale Anmeldenummer: PCT/EP2013/074215
(87) Internationale Veröffentlichungsnummer: WO 2014/082898

(56) Entgegenhaltungen:
- JP-A- 2003 096 004

## Beschreibung

Die vorliegende Erfindung betrifft ein Verfahren zur Herstellung von C₄-Oxygenaten, insbesondere 2-Butanol und Butanon, ausgehend von Ethen und Ethanol.

Butanon, auch als Methyl-Ethyl-Keton oder MEK bezeichnet, ist neben Aceton das wichtigste industriell hergestellte Keton und findet wie dieses als Lösungsmittel in vielen Bereichen Verwendung. Butanon kann durch die direkte Oxidation von n-Buten, z.B. nach dem Wacker-Verfahren, oder als Nebenprodukt der Phenolherstellung aus Benzol erhalten werden. Der weitaus überwiegende Teil (88%) an Butanon wird aber durch die katalytische Dehydrierung von 2-Butanol hergestellt. Dieses sehr wirtschaftliche Verfahren liefert hohe Ausbeuten und zeichnet sich durch eine hohe Lebensdauer des verwendeten Katalysators, einfache Produktabtrennung und einen geringen Energieverbrauch aus. Das für die Butanonherstellung eingesetzte 2-Butanol wird durch die Hydratisierung von n-Buten gebildet, welches aus petrochemisch hergestellten C₄-Raffinaten stammt. (Ullmann's Encyclopedia of Industrial Chemistry, 6. Ausgabe, Band 5, Seiten 725-732). Ein Nachteil dieses Verfahrens ist, dass dafür in der Regel mindestens äquimolare Mengen konzentrierter Schwefelsäure verwendet werden (siehe US 1,912,695).

Die vorliegende Erfindung stellt ein alternatives Verfahren für die Herstellung von 2-Butanol bzw. Butanon bereit, das von Ethen und Ethanol ausgeht. Die radikalische Addition von Alkenen und Alkoholen ist seit langem bekannt (z.B. Methoden der Organischen Chemie, Houben-Weyl, Band VI/lb, 1984, Seiten 654 ff.; Urry et al., J. Am. Chem. Soc., 1954, 76: 450-455). Es sind verschiedene Verfahren beschrieben, bei denen die Radikalbildung durch Zusatz von Peroxiden, durch Strahlung oder durch Verwendung des Alkohols im superkritischen Zustand (erhöhter Druck, erhöhte Temperatur) initiiert wird (Urry et al., *supra;* Kamitanaka et al., Tetrahedron Letters 2007, 48: 8460-8463; JP 2003-096004 A). Diese Verfahren führen häufig zu relativ geringen Ausbeuten. Insbesondere bei verlängerter Reaktionszeit findet bei derartigen Reaktionen oft zunehmend eine Bildung von Oligomeren statt, welche die Gesamtausbeute verringert. Nicht zuletzt aus diesem Grund wurde bisher keines dieser Verfahren zur industriellen Herstellung von 2-Butanol und Butanon eingesetzt.

Die vorliegende Erfindung stellt ein Verfahren zur Herstellung von C₄-Oxygenaten bereit, umfassend die Umsetzung von Ethen mit Ethanol zu 2-Butanol unter Bedingungen, bei denen sich Ethanol im überkritischen Zustand befindet, wobei die Umsetzung von Ethen mit Ethanol bei einer Temperatur im Bereich von 280°C bis 300°C und bei einem Druck im Bereich von 25 MPa bis 30 MPa stattfindet.

Durch dieses Verfahren kann 2-Butanol mit hoher Selektivität und gutem Umsatz erhalten wird. Das Verfahren stellt somit eine echte Alternative zur 2-Butanolherstellung aus n-Buten dar. Dies war überraschend, weil durch die bekannten Verfahren zur Addition von Alkenen und Alkoholen deutlich geringere Umsätze und schlechtere Selektivitäten erzielt wurden. Überraschend war es auch insofern, als mit Ethen ein gasförmiger Ausgangsstoff eingesetzt wird und der Übergang in den überkritischen Zustand somit nicht aus einem homogenen flüssigen Gemisch, sondern aus einem flüssig-gasförmigen Zweikomponentensystem erfolgt.

Das aus Ethen und Ethanol gebildete 2-Butanol kann zu Butanon dehydriert werden. Somit stellt die vorliegende Erfindung ein Verfahren zur Herstellung von Butanon ausgehend von Ethen und Ethanol bereit.

Ethen und Ethanol sind leicht verfügbare, relativ preiswerte Ausgangsstoffe. Ethen wird in großen Mengen petrochemisch gewonnen. Es kann aber z.B. auch aus Ethanol hergestellt werden (Ullmann's Encyclopedia of Industrial Chemistry, 6. Ausgabe, Band 12, Seiten 532-575). Auf diesem Wege ist eine Butanonherstellung möglich, die vollständig auf nachwachsenden Rohstoffen beruht. Weitere Vorteile des Verfahrens sind, dass keine teuren und/oder gefährlichen Reagenzien, wie z.B. Peroxide, Strahlungsquellen, Schwefelsäure oder weitere Lösungsmittel, eingesetzt werden müssen und die Umsetzung höchst atomökonomisch abläuft.

Sowohl die Umsetzung von Ethen und Ethanol zum Ethan als auch die Dehydrierung des 2-Butanols zum Butanon lassen sich in technischem Maßstab problemlos durchführen und liefern die jeweiligen Produkte mit hoher Selektivität und guten Ausbeuten.

Für die erfindungsgemäße Herstellung von 2-Butanol kann Ethanol, bezogen auf Ethen, im Überschuss, im Unterschuss oder in einer äquimolaren Menge eingesetzt werden. Es ist vorteilhaft, mit einem Überschuss an Ethanol zu arbeiten. Bevorzugt liegt das molare Verhältnis des eingesetzten Ethens zum eingesetzten Ethanol im Bereich von 1:2 bis 1:1000, mehr bevorzugt im Bereich von 1:2 bis 1:500, noch mehr bevorzugt im Bereich von 1:2 bis 1:200 und am meisten bevorzugt im Bereich von 1:20 bis 1:150.

Das erfindungsgemäße Verfahren und insbesondere die erfindungsgemäße Herstellung von 2-Butanol kann in Anwesenheit oder in weitgehender oder vollständiger Abwesenheit weiterer Lösungsmittel erfolgen. Bevorzugt wird kein weiteres Lösungsmittel zugesetzt. Die erfindungsgemäße Herstellung von 2-Butanol kann in Anwesenheit oder in weitgehender oder vollständiger Abwesenheit eines Katalysators oder Initiators, z.B. eines Radikalinitiators wie Peroxid, erfolgen. Bevorzugt wird kein Katalysator oder Initiator zugesetzt. Weitgehende Abwesenheit bedeutet, dass die Konzentration weiterer Lösungsmittel, Katalysatoren bzw. Initiatoren, bezogen auf das Gesamtgewicht des Reaktionsgemischs, weniger als 1 g/kg (< 1000 ppm), insbesondere weniger 0,1 g/kg (< 100 ppm), beträgt.

Erfindungsgemäß wird die Umsetzung von Ethen mit Ethanol bei Bedingungen durchgeführt, bei denen Ethanol im überkritischen Zustand vorliegt, d.h. oberhalb des kritischen Punkts. Der kritische Punkt von Ethanol liegt bei einer Temperatur von 241 °C und einem Druck von 6,3 MPa. Im überkritischen Zustand sind Flüssigkeit und Gas nicht mehr voneinander zu unterscheiden, die Substanz liegt als sogenanntes überkritisches Fluid vor. Die Dichte des überkritischen Fluids kann durch Druckerhöhung nicht weiter erhöht werden. Sie entspricht im Wesentlichen der Dichte im flüssigen Zustand. Demgegenüber entspricht aber die Viskosität des überkritischen Fluids im Wesentlichen der Viskosität im gasförmigen Zustand. Das überkritische Fluid weist somit eine relativ hohe Dichte, niedrige Viskosität und hohe Dispergierbarkeit auf und ist ein optimales Lösungsmittel.

Zur Herstellung von 2-Butanol wird Ethanol bzw. das Reaktionsgemisch aus Ethanol und Ethen auf eine Temperatur von 241 °C oder mehr erhitzt und einem Druck von 6,3 MPa oder mehr ausgesetzt. Beispielsweise kann man dazu das Ethanol in einem druckfesten Gefäß vorlegen, mit Ethen beaufschlagen und das Gemisch unter Eigendruck erwärmen bis Bedingungen erreicht sind, bei denen sich Ethanol im überkritischen Zustand befindet. Erfindungsgemäß wird bei Reaktionstemperaturen im Bereich von 280-300°C gearbeitet. Der dabei angelegte Druck liegt im Bereich von 25 bis 30 MPa.

Die Reaktionsdauer hängt naturgemäß von den gewählten Bedingungen und dem gewünschten Umsatz ab. Bei der erfindungsgemäßen Umsetzung von Ethen und Ethanol wird das Reaktionsgemisch typischerweise über eine Dauer im Bereich von 10 Sekunden bis 3 Stunden der Reaktionstemperatur (d.h. ≥ 241 °C) und dem Reaktionsdruck (d.h. ≥ 6,3 MPa) ausgesetzt. Falls gewünscht, kann dieser Vorgang zur Erhöhung des Umsatzes und damit der Ausbeute einmal, zweimal, dreimal, viermal oder noch häufiger wiederholt werden. In der Regel wird die Reaktion soweit geführt, dass der im Unterschuss eingesetzte Reaktand, bei dem es sich vorzugsweise um Ethen handelt, zu wenigstens 80%, insbesondere zu wenigstens 90% umgesetzt ist.

Die erfindungsgemäße Umsetzung von Ethen mit Ethanol kann absatzweise durchgeführt werden (Batch-Fahrweise), d.h. Ethanol und Ethen werden im gewünschten Molverhältnis in einem geeigneten Reaktor vorgelegt, auf die gewünschten Reaktionsbedingungen gebracht und bis zu dem gewünschten Umsatz unter Reaktionsbedingungen gehalten.

Die erfindungsgemäße Umsetzung von Ethen mit Ethanol kann auch in der sogenannten Semi-Batch-Fahrweise durchgeführt werden, d.h. die Hauptmenge, in der Regel wenigstens 80%, insbesondere wenigstens 90%, eines oder beider Reaktanden, wird kontinuierlich oder portionsweise über einen längeren Zeitraum, in der Regel wenigstens 50% der gesamten Umsatzdauer, unter Reaktionsbedingungen in den Reaktor gegeben. Zum Beispiel kann wenigstens 80%, insbesondere wenigstens 90%, der eingesetzten Verbindung des Ethanols gegebenenfalls zusammen mit einer Teilmenge des Ethens vorlegt und wenigstens 80%, insbesondere wenigstens 90% des eingesetzten Ethens der Umsetzung unter Reaktionsbedingungen zuführt werden.

Die erfindungsgemäße Umsetzung von Ethen mit Ethanol kann auch kontinuierlich durchgeführt werden, d.h. Ethen und Ethanol werden im gewünschten Molverhältnis kontinuierlich in eine Reaktionszone eingespeist und das Reaktionsgemisch wird kontinuierlich der Reaktionszone entnommen. Die Rate, mit der Ethen und Ethanol der Reaktionszone zugeführt werden, richtet sich nach der gewünschten Verweilzeit, die ihrerseits in bekannter Weise von der Reaktorgeometrie abhängt und der oben angegebenen Umsetzungsdauer entspricht.

Die erfindungsgemäße Umsetzung von Ethen mit Ethanol kann grundsätzlich in allen Reaktoren durchgeführt werden, welche für die gewählten Reaktionsbedingungen geeignet sind, vorzugsweise in Autoklaven, die Vorrichtungen zur Durchmischung der Reaktanden aufweisen können, oder in Reaktionsrohren.

Um das Molverhältnis von Ethen zu Ethanol während der Umsetzung relativ stabil zu halten und gleichzeitig eine besonders effiziente Reaktionsführung zu ermöglichen, hat es sich als vorteilhaft erwiesen, wenn man wenigstens 80%, insbesondere wenigstens 90% des eingesetzten Ethanols gegebenenfalls zusammen mit einer Teilmenge des Ethens vorlegt und wenigstens 80%, insbesondere wenigstens 90% des eingesetzten Ethens der Umsetzung unter Reaktionsbedingungen zuführt. Die Zugabe des Ethens kann portionsweise oder kontinuierlich erfolgen, wobei eine kontinuierliche Zugabe bevorzugt ist. Die Geschwindigkeit, mit der man das Ethen zuführt, wird dabei vorzugweise so gewählt, dass das Molverhältnis des in die Reaktionszone bzw. den Reaktor eingespeisten, noch nicht abreagierten Ethens zu dem in der Reaktionszone befindlichen Ethanol während der Umsetzung im Bereich von 1:2 bis 1:1000, mehr bevorzugt im Bereich von 1:2 bis 1:500, besonders bevorzugt im Bereich von 1:2 zu 1:200 und insbesondere im Bereich von 1:20 bis 1:150 liegt. Bei einer kontinuierlichen Reaktionsführung wird man daher vorzugsweise Ethen und Ethanol in den zuvor genannten molaren Verhältnissen dem Reaktor bzw. der Reaktionszone zuführen.

Das durch die Umsetzung von Ethen und Ethanol erhaltene Reaktionsgemisch kann in an sich bekannter Weise aufgearbeitet werden oder, gegebenenfalls nach Entfernung des Ethanols, unmittelbar als solches unter Bildung von Butanon dehydriert werden. Das bei der Umsetzung von Ethen mit Ethanol anfallende Reaktionsgemisch kann, beispielsweise extraktiv oder destillativ oder durch eine Kombination dieser Maßnahmen aufgearbeitet werden. In einer Ausführungsform des erfindungsgemäßen Verfahrens arbeitet man das bei der Umsetzung von Ethen mit Ethanol anfallende Reaktionsgemisch destillativ auf, wobei man 2-Butanol als Mittelfraktion von Leicht- und Schwersiedern abtrennt. Sofern mit einem Überschuss an Ethanol gearbeitet wird, kann die Leichtsieder-Fraktion, die überwiegend aus Ethanol besteht, in den Prozess zurückgeführt werden. In der Regel wird man vor der Dehydrierung das Ethanol weitgehend entfernen, so dass der Ethanolanteil in dem zur Dehydrierung eingesetzten 2-Butanolhaltigen Produkt weniger als 20 Gew.-%, insbesondere nicht mehr als 10 Gew.-%, bezogen auf die Gesamtmenge des Produkts, beträgt.

Das bei der Umsetzung von Ethen mit Ethanol anfallende 2-Butanol kann mit bekannten Verfahren zum Butanon dehydriert werden. Derartige Verfahren zur oxidativen Dehydrierung sind beispielsweise in den US-Patentschriften 5,723,679 und 4,380,673 beschrieben.

Die Dehydrierung von 2-Butanol erfolgt zweckmäßigerweise an einem geeigneten Katalysator. Dabei handelt es sich üblicherweise um Katalysatoren, die wenigsten ein Aktivmetall enthalten, das aus den Gruppen I, II, VI, VII und VIII des Periodensystems (CAS-Version) und insbesondere unter Kupfer, Magnesium, Nickel und Zink ausgewählt ist. Geeignete Katalysatoren sind beispielsweise in den US-Patentschriften 5,723,679 und 4,380,673 beschrieben. Die Dehydrierung kann in der Gasphase, z.B. bei Temperaturen von 250°C bis 550°C, insbesondere bei 300°C bis 400°C, erfolgen. Alternativ kann die Dehydrierung in der Flüssigphase erfolgen, z.B. bei Temperaturen von 170°C bis 230°C und unter Verwendung von C₁₂-C₂₀ paraffinischen Kohlenwasserstoffen als Lösungsmittel für 2-Butanol. Die Dehydrierung kann sowohl kontinuierlich als auch diskontinuierlich ausgestaltet werden, wobei die kontinuierliche Durchführung des Verfahrens bevorzugt ist.

Die Erfindung wird anhand der folgenden Beispiele näher erläutert:

### Beispiel 1: Synthese von 2-Butanol aus Ethen und Ethanol in Batch-Fahrweise

150 g Ethanol wurden im Autoklav (300-ml-Autoklav HC 700 bar) vorgelegt und eine Dichtigkeitsprüfung mit Stickstoff durchgeführt. Anschließend wurden 0,91 g Ethen aufgepresst und das Reaktionsgemisch bis zu einer Temperatur von 290°C erhitzt, wobei ein Druck von 27 MPa erreicht wurde. Nach 2 Stunden Reaktionsdauer unter diesen Bedingungen wurde der Autoklav auf Raumtemperatur abgekühlt und entspannt. Das erhaltene Produkt wurde mittels Gaschromatographie unter Verwendung von 2-Nonanon als internem Standard analysiert. Es enthielt 1,4 g 2-Butanol, was bezogen auf das eingesetzte Ethen einem Umsatz von 58% entspricht. Die Selektivität (umsatzbezogene Ausbeute) bezüglich 2-Butanol betrug 90%.

### Beispiel 2: Synthese von 2-Butanol aus Ethanol und Ethylen in Semi-Batch-Fahrweise

150 g Ethanol wurden im Autoklav (300-ml-Autoklav HC 700 bar) vorgelegt. Anschließend wurden 2 g Ethen aufgepresst und das Reaktionsgemisch bis zu einer Temperatur von 290°C erhitzt, wobei ein Druck von 27 MPa erreicht wurde. Nach 2 Stunden Reaktionsdauer unter diesen Bedingungen wurde der Autoklav auf Raumtemperatur abgekühlt und entspannt. Das Aufpressen von 2 g Ethen zusammen mit der anschließenden Reaktion bei 290°C und 27 MPa wurde viermal wiederholt. Das erhaltene Produkt wurde mittels Gaschromatographie unter Verwendung von 2-Nonanon als internem Standard analysiert. Die Selektivität (umsatzbezogene Ausbeute) bezüglich 2-Butanol betrug 73%.

## Patentansprüche

1. Verfahren zur Herstellung von C₄-Oxygenaten, umfassend die Umsetzung von Ethen mit Ethanol zu 2-Butanol unter Bedingungen, bei denen sich Ethanol im überkritischen Zustand befindet, wobei die Umsetzung von Ethen mit Ethanol bei einer Temperatur im Bereich von 280°C bis 300°C und bei einem Druck im Bereich von 25 MPa bis 30 MPa stattfindet.

2. Verfahren nach Anspruch 1, bei dem man Ethanol in einem druckfesten Gefäß vorlegt, mit Ethen beaufschlagt und das Gemisch unter Eigendruck erwärmt.

3. Verfahren nach Anspruch 1 oder 2, wobei das Ethen und das Ethanol in einem molaren Verhältnis im Bereich von 1:2 bis 1:200 eingesetzt werden.

4. Verfahren nach einem der Ansprüche 1 bis 3, wobei die Umsetzung von Ethen mit Ethanol ohne Zusatz eines Radikalstarters stattfindet.

5. Verfahren nach einem der Ansprüche 1 bis 4, ferner umfassend die Dehydrierung des 2-Butanols unter Bildung von Butanon.

## Claims

1. A process for preparing C₄-oxygenates, which comprises reaction of ethene with ethanol to form 2-butanol under conditions under which ethanol is present in the supercritical state wherein the reaction of ethene with ethanol takes place at a temperature in the range from 280°C to 300°C and at a pressure in the range from 25 MPa to 30 MPa.

2. The process according to claim 1, wherein ethanol is placed in a pressure-rated vessel, ethene is introduced and the mixture is heated under autogenous pressure.

3. The process according to claim 1 or 2, wherein the ethene and the ethanol are used in a molar ratio in the range from 1:2 to 1:200.

4. The process according to any of claims 1 to 3, wherein the reaction of ethene with ethanol takes place without addition of a free-radical initiator.

5. The process according to any of claims 1 to 4 which further comprises dehydrogenation of the 2-butanol to form butanone.

## Revendications

1. Procédé de fabrication d'oxygénats en C₄, comprenant la mise en réaction d'éthène avec de l'éthanol pour former du 2-butanol dans des conditions dans lesquelles l'éthanol se trouve dans un état sur-critique, la réaction de l'éthène avec l'éthanol ayant lieu à une température dans une plage de 280°C à 300°C et à une pression dans une plage de 25 MPa à 30 MPa.

2. Procédé selon la revendication 1, dans lequel on place l'éthanol dans un récipient résistant à la pression, on le met en présence d'éthène et le mélange est chauffé à la pression propre.

3. Procédé selon la revendication 1 ou 2, dans lequel l'éthène et l'éthanol sont utilisés dans un rapport molaire dans une plage de 1:2 à 1:200.

4. Procédé selon l'une quelconque des revendications 1 à 3, dans lequel la réaction de l'éthène avec l'éthanol a lieu sans ajouter d'amorceur radicalaire.

5. Procédé selon l'une quelconque des revendications 1 à 4, comprenant en outre la déshydratation du 2-butanol avec formation de butanone.
